# Europäisches Patentamt

# European Patent Office

# Office européen des brevets

(19)

(11) Veröffentlichungsnummer: **0 056 065**
**B1**

(12) ## EUROPÄISCHE PATENTSCHRIFT

(45) Veröffentlichungstag der Patentschrift:
04.04.84

(51) Int. Cl.³: **A 61 K 9/20, A 61 K 31/557**

(21) Anmeldenummer: **81100140.3**

(22) Anmeldetag: **10.01.81**

(54) **Stabilisierte, lokal anwendbare Prostaglandintabletten mit regulierter Auflösungsgeschwindigkeit und Verfahren zu der Herstellung.**

(43) Veröffentlichungstag der Anmeldung:
21.07.82 Patentblatt 82/29

(45) Bekanntmachung des Hinweises auf die Patenterteilung:
04.04.84 Patentblatt 84/14

(84) Benannte Vertragsstaaten:
BE CH DE FR GB IT LI NL SE

(56) Entgegenhaltungen:
DE - A - 2 353 797
DE - A - 2 515 001
DE - A - 2 719 690

CHEMICAL ABSTRACTS, Band 86, Nr. 12, 21. März 1977, Seite 401, Nr. 78688a

(73) Patentinhaber: **CHINOIN Gyogyszer és Vegyészeti Termékek Gyára RT., To utca 1-5, H-1045 Budapest V (HU)**

(72) Erfinder: **David, Agoston, Dr., Battyány u. 12, H-1015 Budapest (HU)**
Erfinder: **Horváth, Tibor, Dr., Bethlen G. u. 9, H-1153 Budapest (HU)**
Erfinder: **Kiss, Csaba, Dr., Bakator u. 11, H-1118 Budapest (HU)**
Erfinder: **Nagy, Gábor, Dr., Erdöalja u. 7, H-1037 Budapest (HU)**
Erfinder: **Simon, Kálmán, Dr., Szentpétery u. 13, H-1115 Budapest (HU)**
Erfinder: **Simonidesz, Ilona, Dr. geb. Vermes, Fenyö u. 13, H-1016 Budapest (HU)**
Erfinder: **Udvardi, Agnes, Dipl.-Ing. Chem., Sziklai S. u. 7, H-1012 Budapest (HU)**
Erfinder: **Virág, Sándor, Dr., Rákoczi u. 11, H-1088 Budapest (HU)**

(74) Vertreter: **Lotterhos, Hans Walter, Dr.-Ing., Lichtensteinstrasse 3, D-6000 Frankfurt am Main 1 (DE)**

## Stabilisierte, lokal anwendbare Prostaglandintabletten mit regulierter Auflösungsgeschwindigkeit und Verfahren zu der Herstellung

Die vorliegende Erfindung betrifft stabilisierte, lokal anwendbare Prostaglandintabletten mit regulierter Auflösungsgeschwindigkeit und ein Verfahren zur Herstellung dieser Tabletten.

Es ist bekannt, daß die Prostaglandine vor allem in der Gynäkologie aufgrund der Erfahrungen von N. Winquist und seinen Mitarbeitern (Lancet, 889, 1970) angewandt worden sind. Auch in Ungarn sind derartige Forschungen durchgeführt worden (Orvosi Hetilap 113, 919—927 (1972), Magyar Nöorvosok Lapja 37, 97—103 (1974)), deren Ergebnisse dazu führten, daß Prostaglandin in $F_{2\alpha}$-enthaltende Präparate in Form von injizierbaren Lösungen in die Humantherapie eingeführt und insbesondere bei Schwangerschaftsunterbrechungen, Muttermunderweiterungen und um Entbindungen in Gang zu bringen angewandt worden sind. Die Herstellung solcher Injektionslösungen ist in der HU-PS 171 997 beschrieben.

Da die intrauterine, intraaminale und intravenöse Anwendung einen besonderen Aufwand sowie eine große Umsicht erfordert und außerdem für die behandelte Person eine relativ starke Belastung darstellt, bestand schon seit längerer Zeit ein großes Interesse an einer einfacher applizierbaren pharmazeutischen Anwendungsform für Prostaglandinpräparate.

Es ist auch bekannt, daß die oral applizierbaren pharmazeutischen Präparate nur bei Anwendung in relativ hohen Dosen ein klinisches Ergebnis liefern, das infolge dessen in starkem Maße von unerwünschten Nebenwirkungen begleitet ist.

Es ist weiterhin bekannt, in der Gynäkologie Vaginaltabletten und halbfeste pharmazeutische Dosiereinheiten zu benutzen. In diesen Anwendungsformen sind aber die Prostaglandinpräparate wegen der anatomischen und physiologischen Verhältnisse in der Gynäkologie nur schwer einzusetzen, da ein therapeutischer Erfolg nur mit einer wesentlichen Überdosierung zu erzielen ist und eine solche Behandlung mit derartigen Anwendungsformen von einer Reihe von Unsicherheiten begleitet ist.

Eine vorteilhafte Anwendungsform wäre die Sublingual- oder die Zervikaltablette, die am Ort der Behandlung den Wirkstoff gleichmäßig abgibt. Solche Tabletten konnten aber mit den bisher bekannten Methoden aus Prostaglandinen nicht hergestellt werden.

Hindernis für die Herstelung solcher Prostaglandinpräparate war die bekannte Instabilität der Prostaglandine. Bei Lagerung der mittels bekannter Tablettierungsmethoden erhaltenen Zervikal- und Sublingualtabletten an der Luft verlieren die Tabletten ihren Wirkstoff mit der aus Tabelle I ersichtlichen Geschwindigkeit.

Zusammensetzung der für die Untersuchungen der Tabelle I benutzten Tabletten:
2,5 mg $PGF_{2\alpha}$, 24,5 mg Lactose und 1 mg Stearin.

Tabelle I

| Lagertemperatur (°C) | 0 | 30 | 40 | 50 | 60 |
|---|---|---|---|---|---|
| Wirkstoffgehalt nach 30 Tagen (%) | 100 | 90,3 | 85,7 | 67,0 | 37,2 |

Tabletten, die statt Lactose Mannit oder Sorbit und statt Stearin fließendes Paraffin enthalten, sind ebenfalls instabil.

Aus der HU-PS 171 997 ist bekannt, daß die Stabilität von Prostaglandin enthaltenden Injektionspräparaten durch Zusatz von Alkaliacetat wesentlich verbessert werden kann. Die Stabilität von Prostaglandin enthaltenden Tabletten konnte aber auf diese Weise nicht erhöht werden.

Es wurde gefunden, daß man stabile und für die zervikale und die sublinguale Anwendung geeignete Tabletten erhält, wenn man dem zu tablettierenden Gemisch einen Puffer zusetzt, der sicherstellt, daß der unter der Einwirkung der Luftfeuchtigkeit auf der Oberfläche der Tablette gebildete monomolekulare Flüssigkeitsfilm einen schwach sauren pH-Wert (zwischen 3—5) aufweist. Als solche Puffer sind insbesondere Citronensöure/Citrat- und Dialkali-hydrogen-phosphat/Weinsäure-Systeme geeignet.

Die Tabletten könnnen als Füllstoff Zucker, Zuckeralkohole sowie Lactose, Mannit, gegebenenfalls Süßmittel, wie Saccharin, Aromastoffe, wie Cyclodextrin-Aromakomplexe, sowie Formulierungshilfsstoffe enthalten.

Über das Verhältnis der als Formulierungshilfsstoffe verwendeten Stearinsäure und des Erdalkalistearats kann die Geschwindigkeit der Wirkstoffabgabe der Tabletten beeinflußt werden.

Gemäß der Erfindung wird eine Mischung von 0,2—20 Gew.% eines oder mehrerer natürlicher oder synthetischer Prostaglandine mit 0,4—40 Gew.% eines atoxischen Puffersystems, das in dem auf der Oberfläche der festen Phase unter der Einwirkung der Luftfeuchtigkeit gebildeten Flüssigkeitsfilm einen pH-Wert von 3—5 sichert, 1-50 Gew.% Stearinsäure, 0—15 Gew.% Erdalkalistearat, 10—95 Gew.% Lactose und/oder Mannit, 0—15 Gew.% Granulierungshilfsstoffen, 1—30 Gew.% den Zerfall fördernden Stoffen und gewünschtenfalls 0,5—10 Gew.% Geschmackstoffen und Aromastoffen granuliert und unter einem Druck von 49,03—196,13 MPa (500—2000 kp/cm$^2$) zu Tabletten verpreßt.

Es hat sich als besonders vorteilhaft erwiesen, bei dem Pressen der Tabletten einen Enddruck von 78,45—159,91 MPa (800—1600 kp/cm$^2$) anzuwenden, da unter solchen Bedingungen in der Tablette ein Kristallisierungsprozeß stattfindet, der zur Folge hat, daß die in den Tabletten teilweise gebildete heterogene Struktur vom Einzelkristalltyp den Zerfall der Tablette verzögert, wodurch auch die Abgabe des Wirkstoffes langsamer und damit gleichmäßiger wird.

In der Beschreibung ist der Ausdruck Tablette breit auszulegen; unter Tabletten sind, unabhängig von deren Form, alle durch Verpressen erhaltenen Präparate, die die oben angegebene Zusammensetzung haben und unter Anwendung der oben angegebenen Preßdrucke hergestellt worden sind, zu verstehen. Mit anderen Worten: unter Tabletten sind hier Tabletten in der üblichen Scheibenform, in platter Form, mit einer Kugelkappe oder in Stäbchenform, die auch als eine Vervielfachung der üblichen Tablette in der Längsrichtung betrachtet werden können, zu verstehen.

Die Stäbchenform ist besonders für die zervikale Anwendung geeignet. Die Zervikaltablette ist vorzugsweise 10—15 mm lang, hat einen Durchmesser von 3 mm und gibt ihren Wirkstoff in der ganzen Länge des Zervikalkanals gleichmäßig ab. Dies ist vorteilhaft für die Patientin und für den Arzt, der vor der Behandlung nicht die übliche Tablettenserie mit der nötigen Dosis an klassischen Tabletten mit geringerem Wirkstoffgehalt zusammenstellen muß.

In der nachfolgenden Tabelle sind die Ergebnisse eines Röntgendiffraktions-Vergleichsversuches mit einer gemäß Erfindung erhaltenen Tablette und mit der Lactose, die den überwiegenden Teil der Tablette ausmacht, zusammengestellt.

Der Versuch wurde mit Phillips Pulverdiffraktometer, durch eine mit Nickel filtrierte CuK$_\alpha$-Bestrahlung, mit einer 40 kV, 20 mA Induktion in einem Winkelbereich von 3—40° $\theta$ durchgeführt.

Die Tablette gemäß der Erfindung wurde nach Beispiel 1 hergestellt.

Tabelle II

| 2 $\theta$ (°) | Relative Spitzenhöhe (%) | |
|---|---|---|
| | Tablette nach Beispiel 1 | Lactose |
| 12,3 | 28 | — |
| 14,7 | — | 5 |
| 16,2 | — | 25 |
| 16,4 | 25 | — |
| 17,2 | 8 | — |
| 17,8 | — | 5 |
| 19,2 | 37 | — |
| 19,5 | 51 | — |
| 19,6 | — | 100 |
| 20,0 | 65 | — |
| 20,9 | 20 | — |
| 21,2 | — | 30 |
| 21,3 | 26 | — |
| 22,7 | 11 | — |
| 23,0 | — | 5 |
| 23,6 | 18 | 20 |
| 25,4 | — | 15 |

3

Tabelle II (Fortsetzung)

| $2\theta$ | Relative Spitzenhöhe (%) | |
| --- | --- | --- |
| (°) | Tablette nach Beispiel 1 | Lactose |
| 25,7 | 11 | — |
| 27,5 | 8 | 10 |
| 28,1 | — | 10 |
| 33,2 | 6 | — |
| 34,6 | 7 | — |
| 34,9 | 7 | — |
| 36,3 | 6 | — |
| 37,0 | 8 | — |
| 37,6 | 10 | — |

Klinische Testergebnisse mit den 2,5 mg PGF$_{2\alpha}$-Wirkstoff enthaltenden Zervikaltabletten der Erfindung:

| Maß der Muttermunderweiterung (mm) | 3—4 | 4—5 | 5—6 | >6 |
| --- | --- | --- | --- | --- |
| Zahl der Fälle | 46 | 74 | 78 | 21 |
| % | 21 | 34 | 36 | 10 |

| Weite des Muttermundes nach 90 Minuten (in Hegar Einheiten) | 7—8 | 8—9 | 9—10 | >10 |
| --- | --- | --- | --- | --- |
| Zahl der Fälle | 41 | 80 | 76 | 20 |
| % | 19 | 37 | 35 | 9 |

| | Nebenwirkungen | | | |
| --- | --- | --- | --- | --- |
| | Schmerz | Brechreiz | Erbrechen | Kopfschmerz oder andere Schmerzen |
| Zahl der Fälle | 25 | 3 | | 4 |
| % | 11,4 | 1,4 | | 1,9 |

Der Muttermund war in jedem Fall so aufgelockert, daß die Schwangerschaftsunterbrechung ohne weitere Erweiterung durchführbar war. In 32 Fällen war der Abortus nach 90 Minuten schon in dem Muttermund. Eine 3 Monate nach der Schwangerschaftsunterbrechung durchgeführte Kontrolle zeigte, daß der Muttermund in allen Fällen funktionell intakt war.

Der Testversuch wurde in folgender Weise durchgeführt:

In einen für die intrauterine Anwendung des Pessariums geeigneten Sontagh-Applikator wurden 4—7 Tabletten gegeben, der Applikator bis zu einer Tiefe von 15 mm in den Zervikalkanal eingeführt und dann die Tabletten aus dem Applikator hinausgeschoben. Ein Teil der Tabletten konnte in die Gebärmutterhöhlung gekommen sein. Die Weite des Muttermundes wurde unmittelbar nach der Auflegung der Tabletten und dann nach 60 bzw. 90 Minuten durch eine konische Sonde gemessen. Die nach 60 bzw. 90 Minuten erhaltenen Werte waren gleich.

Die weiteren Einzelheiten der Erfindung sind den nachfolgenden Beispielen zu entnehmen. Hieraus sind keine Beschränkungen herzuleiten.

### Beispiel 1

Es wurden Zervikaltabletten folgender Zusammensetzung hergestellt:

| | |
|---|---|
| PGF$_{2\alpha}$ | 2,5 mg |
| Lactose | 22,35 mg |
| Polyvinylpyrrolidon | 1,40 mg |
| Natriumcitrat | 1,25 mg |
| Citronensäure | 1,25 mg |
| Stearinsäure | 1,0 mg |
| Calciumstearat | 0,25 mg |
| | 30,00 mg |

Der Wirkstoff wurde in der 1,6-fachen Äthanolmenge gelöst, die Lösung mit Wasser auf das doppelte Volumen verdünnt und das homogene Pulvergemisch der übrigen festen Komponenten mit dieser Lösung granuliert. Das Granulat wurde getrocknet und erneut granuliert. Die Teilchengröße des Granulates wurde so eingestellt, daß 20% des Granulates eine Teilchengröße von weniger als 0,63 mm aufwiesen und das Volumengewicht etwa 0,5 g/cm³ betrug.

Die Tablettierung wurde mit einem Werkzeug von einem Durchmesser von 3 mm mit einem Preßdruck von 78,5—157 MPa durchgeführt.

Die Festigkeit der erhaltenen Tabletten betrug — mit einem Erweka Gerät gemessen — 2,3—2,8 kg. Die Tabletten lösten sich in destilliertem Wasser bei 37°C innerhalb von 18—20 Minuten auf.

Die Stabilität (Lagerfähigkeit) der Tabletten an der Luft wurde 30 Tage bei verschiedenen Temperaturen beobachtet und der Wirkstoffgehalt bestimmt, wobei folgende Ergebnisse erhalten wurden:

| Lagerungstemperatur (°C): | 0 | 30 | 40 | 50 | 60 |
|---|---|---|---|---|---|
| Wirkstoffgehalt (%): | 100 | 99,0 | 95,6 | 89,5 | 74,1 |

Aus den erhaltenen Angaben kann mittels der prediktionskinetischen Methode (Gyógyszerészet, 18, 81—90 (1974)) berechnet werden, daß in einer Zervikaltablette bei einer Lagerung bei 20°C der Wirkstoffgehalt nach 2 Jahren von 2,5 mg höchstens auf 2,47 mg sinkt, die Stabilität der Tablette gemäß der Erfindung wird also verdoppelt.

### Beispiel 2

Es wurde eine Sublingualtablette folgender Zusammensetzung hergestellt:

| | |
|---|---|
| PGF$_{2\alpha}$ | 0,2 mg |
| Lactose | 42,98 mg |
| Polyvinylpyrrolidon | 2,33 mg |
| Natriumcitrat | 0,1 mg |
| Citronensäure | 0,1 mg |
| Stearinsäure | 1,67 mg |
| Calciumstearat | 0,42 mg |
| Aroma-$\beta$-Cyclodextrin-inklusionskomplex | 1,20 mg |
| Saccharin | 1,0 mg |
| | 50,00 mg |

PGF$_{2\alpha}$ wurde in der 15-fachen Äthanolmenge aufgelöst, die Lösung mit Wasser auf das doppelte Volumen verdünnt und das homogene Pulvergemisch von den übrigen festen Komponenten — mit Ausnahme des Aroma-$\beta$-Cyclodextrin-inklusionskomplexes — mit der Lösung granuliert. Das Granulat wurde getrocknet, auf eine Teilchengröße von 0,32 mm vermahlen, der Inklusionskomplex zugemischt und das Gemisch mit einer Vorrichtung eines Durchmessers von 5 mm bei einem Enddruck von 58,8 MPa zu Tabletten verpreßt. Die Festigkeit der fertigen Tabletten betrug — mit einem Erweka Gerät gemessen — 2,5 kg.

Als $\beta$-Cyclodextrin-inklusionskomplex können Vanille- oder andere Aromakomplexe verwendet werden. Falls kein Aromakomplex verwendet wird, erhöht man die Menge der Lactose um 1,20 mg.

### Beispiel 3

50 g PGF$_{2\alpha}$ löste man in 350 ml Äthanol und verdünnte die Lösung mit 150 ml Wasser. 85 g Lactose, 65 g Mannit, 11 g Stearinsäure, 15 g Magnesiumstearat, 16,5 g Dinatriumhydrogen-phosphat, 8,5 g Weinsäure, 15 g Polyvinylalkohol wurden pulverisiert und das erhaltene feine homogenisierte Pulvergemisch mit der Lösung granuliert. Das Granulat wurde getrocknet und erneut granuliert. Aus dem

Granulat preßte man bei einer 50 mg Einstellung 5000 Zervikaltabletten mit einem Preßenddruck von 118 MPa.

Beispiel 4

20 g kristallines 15-Methyl-PGF$_{2\alpha}$ wurden in einem Pulververmischer mit hoher Drehzahl mit 2300 g wasserfreier Lactose homogenisiert. Dem erhaltenen Homogenisat mischte man ein zuvor homogenisiertes Pulvergemisch von 10 g Natriumcitrat, 7 g Citronensäure, 3 g Weinsäure, 167 g Stearinsäure, 42 g Calciumstearat, 100 g Saccharin, 2000 g wasserfreier Lactose und 120 g $\beta$-Cyclodextrin-inklusionskomplex zu, stellte den Wassergehalt des Gemisches auf 1,8—2,2% ein und verpreßte es bei einer Einstellung von 48 mg und einem Preßenddruck von 98,1 MPa zu 100 000 Stück Sublingualtabletten.

Beispiel 5

Man löste 35 g PGF$_{2\alpha}$ und 15 g 15-Methyl-PGF$_{2\alpha}$ in 400 ml Äthanol, verdünnte die Lösung mit 400 ml Wasser und sprühte sie auf ein gründlich pulverisiertes und homogenisiertes Gemisch von 450 g Lactose, 28 g Polyvinylpyrrolidon, 125 g Natriumcitrat, 125 g Citronensäure und 250 g Stearinsäure. Die Feuchtigkeit des Gemisches wurde auf 0,8—1,5% eingestellt und bei einer Einstellung von 60 mg und einem Preßenddruck von 157 MPa zu Zervikaltabletten eines Durchmessers von 2,5 mm verpreßt.

Klinische Testversuche mit den Zervikaltabletten nach Beispiel 3 und 5 gaben ähnliche Resultate wie die Tabletten nach Beispiel 1.

Sublingualtabletten nach Beispiel 2 und 4 wurden gebärenden Frauen verabreicht, wodurch die Geburt erleichtert wurde, was auf die Tabletten zurückzuführen war. Der Zervikalkanal wurde aufgelockert, und die spontanen Gebärkontraktionen nahmen zu. Nach der Geburt hatte die Blutung aus der Gebärmutter infolge der Steigerung der Kontraktionen aufgehört, der Muttermund hatte sich geschlossen, wodurch sich die Gefahr der Nachgeburtinfektion verminderte.

Beispiel 6

Es wurden Vaginaltabletten folgender Zusammensetzung in der oben angegebenen Weise hergestellt:

| | |
|---|---|
| PGF$_{2\alpha}$ | 20,0 mg |
| Lactose | 326,5 mg |
| Polyvinylpolypyrrolidon | 123 mg |
| Natriumcitrat | 12,0 mg |
| Citronensäure | 1,0 mg |
| Magnesiumstearat | 10,0 mg |
| Stearin | 5,0 mg |
| Polyvinylpyrrolidon | 2,5 mg |
| | 500,0 mg/Tablette |

Die Pressung wurde mit einer platten Vorrichtung mit einem Durchmesser von 13 mm durchgeführt.

Beispiel 7

Es wurden Zervikaltabletten folgender Zusammensetzung hergestellt:

| | |
|---|---|
| PGF$_{2\alpha}$ | 2,5 mg |
| Lactose | 22,35 mg |
| Natriumcitrat | 2,50 mg |
| Polyvinylpyrrolidon | 1,40 mg |
| Stearin | 1,00 mg |
| Calciumstearat | 0,25 mg |
| | 30,00 mg/Tablette |

Zur Herstellung der Tablette wurden die pulverförmigen Komponenten homogenisiert, das Pulvergemisch mit einer äthanolischen Lösung von PGF$_{2\alpha}$ granuliert, das Granulat getrocknet und nach erneuter Granulierung bei einer Einstellung von 30 mg zu Tabletten verpreßt.

**Patentansprüche**

1. Lokal anwendbare, stabilisierte Prostaglandin enthaltende Tabletten mit regulierter Auflösungsgeschwindigkeit, dadurch gekennzeichnet, daß sie 0,2—20 Gew.% eines oder mehrerer natürlicher oder synthetischer Prostaglandine, 0,4—40 Gew.% eines atoxischen Puffersystems, das in dem Flüssigkeitsfilm, der sich auf der Oberfläche der festen Phase unter Einwirkung der Luftfeuchtigkeit bildet, einen pH-Wert von 3—5 sichert, 1—50 Gew.% Stearinsäure, 0—15 Gew.% Erdalkalistearat, 10—95 Gew.% Lactose und/oder Mannit, 0—15 Gew.% andere Granulierungshilfsstoffe, 1—30 Gew.% den Zerfall fördernde Stoffe und gewünschtenfalls 0,5—10 Gew.% Geschmackstoffe und Aromastoffe enthalten und unter Anwendung eines Druckes von 49,03—196,13 MPa (500—2000 kp/cm$^3$) tablettiert worden sind.

2. Verfahren zur Herstellung von lokal anwendbaren, stabilisierten Prostaglandin enthaltenden Tabletten mit regulierter Auflösungsgeschwindigkeit, dadurch gekennzeichnet, daß man eine Mischung von 0,2—20 Gew.% eines oder mehrerer natürlicher oder synthetischer Prostaglandine 0,4—40 Gew.% eines atoxischen Puffersystems, das in dem Flüssigkeitsfilm, der sich auf der Oberfläche der festen Phase unter Einwirkung der Luftfeuchtigkeit bildet, einen pH-Wert von 3—5 sichert, 1—50 Gew.% Stearinsäure, 0—15 Gew.% Erdalkalistearat, 10—95 Gew.% Lactose und/oder Mannit, 0—15 Gew.% Granulierungshilfsstoffe, 1—30 Gew.% den Zerfall fördernde Stoffe und gewünschtenfalls 0,5—10 Gew.% Geschmackstoffe und Aromastoffe granuliert und das Granulat unter Anwendung eines Druckes von 49,03—196,13 MPa (500—2000 kp/cm$^2$) zu Tabletten verpreßt.

3. Verfahren nach Anspruch 2, dadurch gekennzeichnet, daß man als Prostaglandin Prostaglandin $F_{2\alpha}$ oder 15-Methylprostaglandin $F_{2\alpha}$ verwendet.

4. Verfahren nach Anspruch 2 oder 3, dadurch gekennzeichnet, daß man als Puffersysteme ein Alkalimetall-citrat/Citronensäure- oder ein Dialkali-hydrogen-phosphat/Weinsäure-System verwendet.

5. Verfahren nach Anspruch 2, 3 oder 4, dadurch gekennzeichnet, daß man als Erdalkalistearat Magnesium- oder Calciumstearat verwendet.

6. Verfahren nach Anspruch 2, 3, 4 oder 5, dadurch gekennzeichnet, daß man als Aromastoff und Geschmackstoff einen $\beta$-Cyclodextrin-inklusionskomplex und Saccharin verwendet.

**Claims**

1. Stabilised prostaglandin-containing tablets for topical application, having a regulated speed of dissolution, characterised in that they contain 0.2 to 20% by weight of one or more natural or synthetic prostaglandins, 0.4 to 40% by weight of a non-toxic buffer system which ensures a pH value of 3 to 5 in the liquid film which forms on the surface of the solid phase under the effect of the moisture in the air, 1 to 50% by weight of stearic acid, 0 to 15% by weight of alkaline earth metal stearate, 10 to 95% by weight of lactose and/or mannitol, 0 to 15% by weight of other granulating excipients, 1 to 30% by weight of disintegrationpromoting substances and, if desired, 0.5 to 10% by weight of flavourings and aromatics, and in that they are formed into tablets by the application of a pressure of 49.03 to 196.13 MPa (500 to 2000 kp/cm$^2$).

2. Process for the preparation of stabilised prostaglandin-containing tablets for topical application having a regulated speed of dissolution, characterised in that a mixture of 0.2 to 20% by weight of one or more natural or synthetic prostaglandins, 0.4 to 40% by weight of a non-toxic buffer system which ensures a pH of 3 to 5 in the liquid film which forms on the surface of the solid phase under the effect of the moisture in the air, 1 to 50% by weight of stearic acid, 0 to 15% by weight of alkaline earth metal stearate, 10 to 95% by weight of lactose and/or mannitol, 0 to 15% by weight of granulating excipients, 1 to 30% by weight of disintegrationpromoting substances and, if desired, 0.5 to 10% by weight of flavourings and aromatics is granulated and the granulate is compressed to form tablets by the application of a pressure of 49.03 to 196.13 MPa (500—2000 kp/cm$^2$).

3. Process as claimed in claim 2, characterised in that prostaglandin $F_{2\alpha}$ or 15-methyl-propstaglandin $F_{2\alpha}$ is used as the prostaglandin.

4. Process as claimed in claim 2 or 3, characterised in that an alkali metal citrate/citric acid system or al dialkali hydrogen phosphate/tartaric acid system is used as the buffer system.

5. Process as claimed in claim 2, 3 oder 4, characterised in that magnesium or calcium stearate is used as the alkaline earth stearate.

6. Process as claimed in claim 2, 3, 4 or 5, characterised in that a $\beta$-cyclodextrin inclusion complex and saccharin is used as the aromatic and flavouring.

**Revendications**

1. Comprimés contenant de la prostaglandine, stabilisés, pour l'application locale, à vitesse de dissolution régularisée, caractérisés en ce qu'ils contiennent de 0,2 à 20% en poids d'une ou plusieurs

7

prostaglandines naturelles ou synthétiques, 0,4 à 40% en poids d'un système tampon non toxique assurant un pH de 3 à 5 dans la pellicule de liquide qui se forme à la surface de la phase solide sous l'action de l'humidité de l'air, 1 à 50% en poids d'acide stéarique, 0 à 15% en poids de stéarate alcalino-terreux, 10 à 95% en poids de lactose et/ou de mannitol, 0 à 15% en poids d'autres produits auxiliaires de mise en granulés, 1 à 30% en poids de substances favorisant la désagrégation et. lorsqu'on le désire, 0,5 à 10% en poids de substances améliorant le goût et substances aromatisantes, et ont été mis à l'état de comprimés sous une pression de 49,03 à 196,13 MPa.

2. Procédé de préparation de comprimés contenant de la prostaglandine, stabilisés, pour l'application locale, à vitesse de dissolution régularisée, caractérisé en ce que l'on met à l'état de granulés un mélange de 0,2 à 20% en poids d'une ou plusieurs prostaglandines naturelles ou synthétiques, 0.4 à 40% en poids d'un système tampon non toxique assurant un pH de 3 à 5 dans la pellicule de liquide qui se forme à la surface de la phase solide sous l'action de l'humidité de l'air, 1 à 50% en poids d'acide stéarique, 0 à 15% en poids de stéarate alcalino-terreux, 10 à 95% en poids de lactose et/ou de mannitol, 0 à 15% en poids de produits auxiliaires de mise en granulés, 1 à 30% de substances facilitant la désagrégation et, lorsqu'on le désire, 0,5 à 10% en poids de substances améliorant le goût et substances aromatisantes, et on met les granulés à l'état de comprimés en appiliquant une pression de 49,03 à 196,13 MPa.

3. Procédé selon la revendication 2, caractérisé en ce que la prostaglandine utilisée est la prostaglandine $F_{2\alpha}$ ou la 15-méthyl-prostaglandine $F_{2\alpha}$.

4. Procédé selon la revendication 2 ou 3, caractérisé en ce que l'on utilise en tant que système tampon un système citrate de métal alcalin/acide citrique ou un système hydrogénophoshate dialcalin/acide tartrique.

5. Procédé selon la revendication 2, 3 ou 4, caractérisé en ce que l'on utilise en tant que stéarate alcalino-terreux le stéarate de magnésium ou de calcium.

6. Procédé selon la revendication 2, 3, 4 ou 5, caractérisé en ce que l'on utilise en tant que substance aromatisante et substance améliorant le goût un complexe d'inclusion de la $\beta$-cyclodextrine et de la saccharine.